# EUROPEAN PATENT APPLICATION

(11) **EP 4 379 388 A1**
(43) Date of publication of application: **05.06.2024**
(21) Application number: 22849007.4
(22) Date of filing: 24.05.2022
(51) Int. Cl.: G01N 35/00

(54) **AUTONOMOUS ANALYSIS DEVICE, DATA PROCESSING DEVICE, DATA PROCESSING METHOD, AND PROGRAM**

(30) Priority: 27.07.2021 JP 2021122570
(71) Applicant: HITACHI HIGH-TECH CORPORATION, Tokyo 105-6409 (JP)
(72) Inventor: GOTO Mayuko, Tokyo 105-6409 (JP); IGUCHI Akihiro, Tokyo 105-6409 (JP); YABUTANI Chie, Tokyo 105-6409 (JP)
(74) Representative: MERH-IP Matias Erny Reichl Hoffmann Patentanwälte PartG mbB
(86) International application number: PCT/JP2022/021289
(87) International publication number: WO 2023/007928

(57) **Abstract**

A purpose of the present invention is to provide an autonomous analysis device with which an operator can easily associate and confirm a reaction course and an illness. In order to achieve this purpose, the present invention provides an autonomous analysis device comprising an analysis unit for analyzing a sample, an operation unit for receiving an operation from an operator, a control unit for controlling the analysis unit on the basis of an input from the operation unit and computing measurement data using a measurement value acquired from the analysis unit, and a display unit for displaying the measurement data computed by the control unit, wherein the control unit causes the display unit to display not only measurement reaction course information that is based on the measurement data of the sample being analyzed, but also reference reaction course information that is designated by the operation unit from among items of reference reaction course information classified in advance for each illness.

## Description

### Technical Field

The present invention relates to an autonomous analysis device.

### Background Art

One of tests that can be measured by an autonomous analysis device is a blood coagulation test. The blood coagulation test is for measuring a blood coagulation capacity of a patient, and items thereof mainly include a prothrombin time (PT), an activated partial thromboplastin time (APTT), and a fibrinogen concentration (Fbg). When a patient sample and a reagent are mixed, a coagulation reaction starts. A coagulation time is measured by monitoring the coagulation reaction course by an amount of change in scattered light or an amount of change in absorbance to detect a coagulation point. In recent years, coagulation waveform analysis (CWA) in which the entire course of the coagulation reaction course of the PT and the APTT is extracted as a coagulation waveform and the coagulation waveform is analyzed has progressed.

As one method of the coagulation waveform analysis, PTL 1 discloses a technique of calculating, based on coagulation reaction rate curve data obtained from coagulation reaction waveform data, a peak width time at a predetermined height on a curve and determining a concentration of a coagulation participation component or coagulation abnormality.

### Citation List

### Patent Literature

PTL 1: JP2019-86517A

### Summary of Invention

### Technical Problem

The CWA includes a coagulation waveform and parameters obtained from the coagulation waveform, such as a coagulation rate obtained by first differentiation of the coagulation waveform, a coagulation acceleration obtained by second differentiation of the coagulation waveform, and a maximum coagulation rate and a maximum coagulation acceleration which are maximum values thereof. The number of devices capable of outputting these parameters is increasing, and in the future, prediction of an illness, prediction of severity of hemorrhage, and prediction for hemostatic management by the CWA can be expected.

However, at present, even when certain measurement data (measurement result, coagulation reaction course, and the like) is stored, the measurement data is automatically deleted or deleted every day by an operator for the purpose of avoiding a risk of data confusing and the like, and past data is not effectively used for prediction of an illness. That is, it is difficult for the operator to determine the similar illness when only the coagulation reaction course is viewed.

An object of the invention is to provide an autonomous analysis device, a data processing device, a data processing method, and a program that process measurement data thereof, by which an operator can easily associate a reaction course with an illness and confirm the illness.

### Solution to Problem

In order to achieve the above object, an autonomous analysis device of the invention includes: an analysis unit configured to analyze a sample; an operation unit configured to receive an operation from an operator; a control unit configured to control the analysis unit based on an input from the operation unit and compute measurement data using a measurement value acquired from the analysis unit; and a display unit configured to display the measurement data computed by the control unit, in which the control unit is configured to cause the display unit to display not only measurement reaction course information based on the measurement data of the sample to be analyzed, but also reference reaction course information designated by the operation unit from among reference reaction course information classified in advance on an illness basis.

### Advantageous Effects of Invention

According to the invention, it is possible to provide an autonomous analysis device, a data processing device, a data processing method, and a program that process measurement data thereof, in which an operator can easily find a similar reaction course between a reaction course of a sample to be analyzed and a reaction course classified according to past illnesses and with which the operator can easily associate a reaction course with an illness and confirm the illness.

### Brief Description of Drawings

[FIG. 1] FIG. 1 is a schematic diagram showing a configuration of an autonomous analysis device according to an embodiment of the invention.
[FIG. 2] FIG. 2 is a functional block diagram of a control device provided in the autonomous analysis device.
[FIG. 3] FIG. 3 is a flowchart showing an example of confirmation of a coagulation reaction course of a target specimen.
[FIG. 4] FIG. 4 is a diagram showing an example of a list screen of a measurement result by the autonomous analysis device.
[FIG. 5] FIG. 5 is a diagram showing an example of a list screen of a coagulation reaction course of a target sample.
[FIG. 6a] FIG. 6a is a diagram showing an example of a screen on which a coagulation reaction course of a measurement item APTT of the target sample and a past coagulation reaction course of the measurement item APTT classified into a congenital hemophilia A are displayed side by side.
[FIG. 6b] FIG. 6b is a diagram showing an example of a screen on which the coagulation reaction course of the measurement item APTT of the target sample and a past coagulation reaction course of a measurement item APTT classified into an APS are displayed side by side.
[FIG. 7a] FIG. 7a is a diagram showing a state in which an overwrite button for displaying the past coagulation reaction course in an overlapping manner with the coagulation reaction course of the target sample is selected on the screen in FIG. 6a.
[FIG. 7b] FIG. 7b is a diagram showing an example of a confirmation screen of overwriting, which is displayed after the screen in FIG. 7a.
[FIG. 7c] FIG. 7c is a diagram showing an example of a screen after the overwriting is executed, which is displayed after the screen in FIG. 7b.
[FIG. 7d] FIG. 7d is a diagram showing an example of a screen when a reaction course display area displaying the overwritten reaction course is scrolled on the screen in FIG. 7c.
[FIG. 8a] FIG. 8a is a diagram showing an example of a screen for prompting classification selection and comment input, which is displayed when the coagulation reaction course of the target sample is stored.
[FIG. 8b] FIG. 8b is a diagram showing another example of the screen for prompting the classification selection and the comment input, which is displayed when the coagulation reaction course of the target sample is stored.
[FIG. 9] FIG. 9 is a diagram showing a state in which a reaction course list edit button in the past reaction course display area is selected on the screen in FIG. 6b.
[FIG. 10a] FIG. 10a is a diagram showing an example of a confirmation screen of reaction course deletion, which is displayed when a delete button corresponding to one of the past reaction courses is selected on a screen transitioned from the screen in FIG. 9.
[FIG. 10b] FIG. 10b is a diagram showing an example of a comment change screen displayed when a comment change button corresponding to one of the past reaction courses is selected on the screen transitioned from the screen in FIG. 9.
[FIG. 10c] FIG. 10c is a diagram showing an example of a classification change screen displayed when a classification change button corresponding to one of the past reaction courses is selected on the screen transitioned from the screen in FIG. 9.
[FIG. 11] FIG. 11 is a diagram showing an example of an operation screen when keys (tabs) assigned to illness classifications are edited.
[FIG. 12] FIG. 12 is a diagram displaying a first derivative curve and a second derivative curve as another example of the list screen of the coagulation reaction course of the target sample.
[FIG. 13a] FIG. 13a is a diagram showing an example of a screen when the past coagulation reaction course of the measurement item APTT classified into the congenital hemophilia A is overwritten to the coagulation reaction course of the measurement item APTT of the target sample.
[FIG. 13b] FIG. 13b is a diagram showing the overwritten first derivative curve by scrolling the reaction course display area of the target sample in FIG. 13a.
[FIG. 13c] FIG. 13c is a diagram showing the overwritten second derivative curve by further scrolling the reaction course display area of the target sample in FIG. 13b.
[FIG. 14] FIG. 14 is a basic configuration diagram of an analysis system including the autonomous analysis device and a data processing device as another embodiment.

### Description of Embodiments

Hereinafter, embodiments of the invention will be described with reference to the drawings.

### (Configuration of Autonomous Analysis Device)

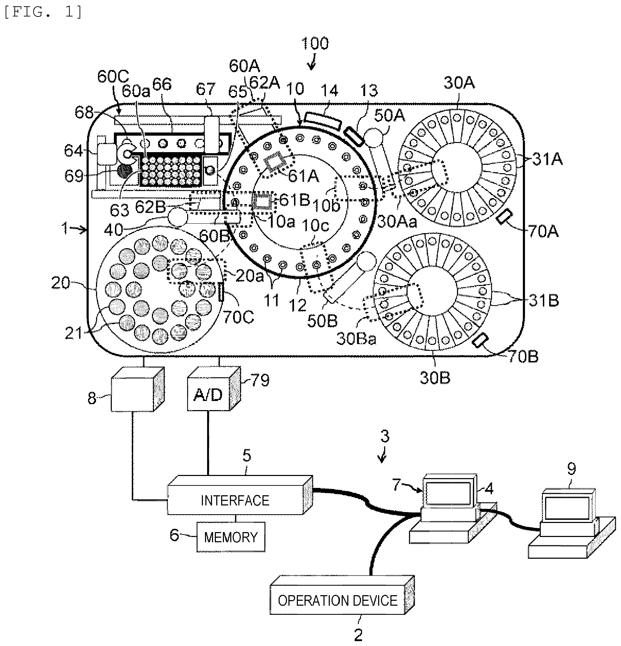

FIG. 1 is a schematic diagram showing a configuration of an autonomous analysis device according to the embodiment of the invention. In the embodiment, a composite autonomous analysis device having functions of biochemical analysis, blood coagulation analysis (blood coagulation fibrinolysis marker, blood coagulation time measurement, and the like), and immune analysis is exemplified as an application target.

An autonomous analysis device 100 shown in FIG. 1 includes an analysis unit 1 (analysis unit) that analyzes a sample, an operation device 2 (operation unit) that receives an operation from an operator and the like, a control device 3 (control unit) that controls the analysis unit 1 based on an input from the operation device 2 and computes measurement data using a measurement value acquired from the analysis unit 1, and a monitor 4 (display unit) that displays and outputs the measurement data computed by the control device 3. The sample to be analyzed by the autonomous analysis device 100 is a specimen such as blood or urine of a patient.

### <Analysis Unit>

The analysis unit 1 includes a reaction disk 10, a sample disk 20, a first reagent disk 30A, a second reagent disk 30B, a sample dispensing mechanism 40, a first reagent dispensing mechanism 50A, a second reagent dispensing mechanism 50B, a first measurement unit 60A to a third measurement unit 60C, and a first reading device 70A to a third reading device 70C.

### -Reaction Disk-

The reaction disk 10 is a disk-shaped unit rotatable around a vertical axis, and holds a large number of reaction containers (reaction cells) 11 made of a transparent material. The reaction container 11 is a container for mixing a sample and a reagent and causing the sample and the reagent to react, and a plurality of reaction containers 11 are annularly arranged on the reaction disk 10. During operation of the autonomous analysis device 100, the reaction containers 11 are kept at a predetermined temperature (for example, about 37°C) in a thermostatic tank 12 of the reaction disk 10. In addition, the reaction disk 10 includes a stirring mechanism 13 and a reaction container cleaning mechanism 14. The stirring mechanism 13 is a device for stirring a liquid contained in the reaction containers 11. The reaction container cleaning mechanism 14 is a device for cleaning the inside of the used reaction containers 11.

### -Sample Disk-

The sample disk 20 is a disk-shaped unit rotatable around a vertical axis, and holds a large number of sample containers 21 containing samples. FIG. 1 exemplifies a configuration in which the sample containers 21 can be concentrically arranged in two rows on the sample disk 20.

### -Reagent Disk-

The first reagent disk 30A is a disk-shaped unit rotatable around a vertical axis, and holds a large number of first reagent bottles 31A. A plurality of first reagent bottles 31A are annularly arranged on the first reagent disk 30A. Similarly, the second reagent disk 30B is a disk-shaped unit rotatable around a vertical axis, and holds a large number of second reagent bottles 31B. A plurality of second reagent bottles 31B are annularly arranged on the second reagent disk 30B. The first reagent bottles 31A and the second reagent bottles 31B contain reagent solutions corresponding to analysis items to be analyzed by the autonomous analysis device 100. Each of the first reagent bottles 31A of the first reagent disk 30A contains, for example, a first reagent or a coagulation reagent used for biochemical and scattering, and each of the second reagent bottles 31B of the second reagent disk 30B contains, for example, a second reagent used for biochemical and scattering.

### -Sample Dispensing Mechanism-

The sample dispensing mechanism 40 has a pipette nozzle, and aspirates and discharges a sample by the pipette nozzle. The sample dispensing mechanism 40 is located between the sample disk 20 and the reaction disk 10. The sample dispensing mechanism 40 aspirates a predetermined amount of the sample from the inside of the sample container 21 at a dispensing position (aspiration position) 20a of the sample disk 20, and discharges the aspirated sample into the inside of the reaction container 11 at the dispensing position (discharge position) 10a of the reaction disk 10.

### -Reagent Dispensing Mechanism-

Each of the first reagent dispensing mechanism 50A and the second reagent dispensing mechanism 50B has a pipette nozzle, and aspirates and discharges a reagent from the pipette nozzle. The first reagent dispensing mechanism 50A is located between the first reagent disk 30A and the reaction disk 10. The second reagent dispensing mechanism 50B is located between the second reagent disk 30B and the reaction disk 10. The first reagent dispensing mechanism 50A aspirates a reagent from the inside of the first reagent bottle 31A corresponding to a test item at a dispensing position (aspiration position) 30Aa of the first reagent disk 30A, and discharges (dispenses) the reagent into the inside of the target reaction container 11 at a dispensing position (discharge position) 10b of the reaction disk 10. Similarly, the second reagent dispensing mechanism 50B aspirates a reagent from the inside of the second reagent bottle 31B corresponding to a test item at a dispensing position (aspiration position) 30Ba of the second reagent disk 30B, and discharges (dispenses) the reagent into the inside of the target reaction container 11 at a dispensing position (discharge position) 10c of the reaction disk 10. The reagent discharged into the reaction container 11 is stirred by the stirring mechanism 13 and mixed with the sample.

### -Measurement Unit-

Each of the first measurement unit 60A to the third measurement unit 60C is a unit that measures a target item for a sample, and includes a light source that irradiates a mixed solution of the sample and the reagent inside the reaction container with light, and a spectrophotometer that detects light transmitted through the mixed solution and outputs a measurement value. The first measurement unit 60A to the third measurement unit 60C measure the reaction of the sample and the reagent inside the reaction container, thereby measuring the target item for the sample.

The first measurement unit 60A and the second measurement unit 60B are measurement units used for a biochemical analysis or an immune analysis. The first measurement unit 60A includes a first light source 61A and a first spectrophotometer 62A. The second measurement unit 60B includes a second light source 61B and a second spectrophotometer 62B. The first light source 61A and the second light source 61B are disposed on an inner peripheral side of the reaction disk 10, and irradiate the reaction containers 11 with light from the inner peripheral side of the reaction disk 10. The first spectrophotometer 62A and the second spectrophotometer 62B are disposed on an outer peripheral side of the reaction disk 10, and respectively face the first light source 61A and the second light source 61B with the annular row of the reaction containers 11 interposed therebetween. The first spectrophotometer 62A and the second spectrophotometer 62B are respectively located on optical axes of the first light source 61A and the second light source 61B. Light emitted from the first light source 61A passes through the reaction containers 11 and is measured by the first spectrophotometer 62A. Similarly, light emitted from the second light source 61B passes through the reaction containers 11 and is measured by the second spectrophotometer 62B. Each reaction container 11 is subjected to photometry for a reaction solution (the mixed solution of the sample and the reagent) contained inside the reaction container 11 every time the reaction container 11 passes through the first measurement unit 60A and the second measurement unit 60B with a rotation operation of the reaction disk 10. The used reaction container 11 is repeatedly used after being cleaned by the reaction container cleaning mechanism 14.

The third measurement unit 60C is a blood coagulation time measurement unit. The third measurement unit 60C includes a reaction container accommodating unit 63, a reaction container transfer mechanism 64, a sample dispensing station 65, a reaction container temperature control block 66, a reagent dispensing mechanism 67, and a measurement channel 68.

In the third measurement unit 60C, a plurality of disposable reaction containers 60a are accommodated in the reaction container accommodating unit 63. The reaction containers 60a are transferred to the sample dispensing station 65 by the reaction container transfer mechanism 64. The sample dispensing station 65 is disposed such that the sample dispensing mechanism 40 is interposed between the sample dispensing station 65 and the sample disk 20. The sample is aspirated from the sample container 21 by the sample dispensing mechanism 40 and discharged (dispensed) into the reaction container 60a of the sample dispensing station 65.

The reaction container 60a to which the sample is dispensed in this manner is transferred from the sample dispensing station 65 to the reaction container temperature control block 66 by the reaction container transfer mechanism 64, and is heated to about 37°C. In addition, the reagent is cooled in the first reagent disk 30A, and the reagent is aspirated from the first reagent bottle 31A corresponding to the test item and discharged to a predetermined empty reaction container 11 disposed in the reaction disk 10 by the first reagent dispensing mechanism 50A, and heated to about 37°C.

After a certain period of time elapses, the reagent kept warm inside the reaction container 11 is aspirated by the reagent dispensing mechanism 67 having a reagent heating function, and further heated to about 40°C by the reagent dispensing mechanism 67. During this period, the reaction container 60a in which the temperature of the sample is kept at about 37°C is transferred to any measurement channel 68 of the third measurement unit 60C by the reaction container transfer mechanism 64. The third measurement unit 60C includes a plurality of measurement channels 68 each having a light source and a spectrophotometer. Thereafter, the warmed reagent is discharged (dispensed) into the reaction container 60a of the measurement channel 68 by the reagent dispensing mechanism 67, and a blood coagulation reaction between the sample and the reagent starts inside the reaction container 60a.

In the measurement channel 68, after the reagent is dispensed into the reaction container 60a, measurement data is output from the spectrophotometer at a predetermined time interval (for example, a cycle of 0.1 seconds) . When the measurement is completed, the used reaction container 60a is transferred by the reaction container transfer mechanism 64 and discarded in a reaction container discarding unit 69.

As described above, in the first measurement unit 60A to the third measurement unit 60C, the scattered light is photoelectrically converted by the spectrophotometer, and a measurement signal (analog signal) having a magnitude proportional to a scattered light intensity is output from the spectrophotometer. The measurement signal is converted into a digital signal by an A/D converter 79 and input to the control device 3.

### -Reading Device-

The first reading device 70A to the third reading device 70C are devices that read identification data attached to containers. A bar code, an RFID, and the like can be adopted as the identification data. In this example, an example using a bar code will be described. That is, the first reading device 70A to the third reading device 70C are bar code readers.

The first reading device 70A reads a bar code attached to the first reagent bottle 31A at the time of reagent registration. The identification data of the reagent read by the first reading device 70A and position data in the first reagent disk 30A of the first reagent bottle 31A to which the identification data is attached are transmitted to the control device 3, and are stored in the memory 6.

The second reading device 70B reads the bar code attached to the second reagent bottle 31B at the time of reagent registration. Similarly to the first reading device 70A, the identification data of the reagent read by the second reading device 70B and position data in the second reagent disk 30B of the second reagent bottle 31B to which the identification data is attached are also transmitted to the control device 3, and are stored in the memory 6.

The third reading device 70C reads the bar code attached to the sample container 21 at the time of sample registration. Data such as a sample ID, a patient ID, and a sample type is converted into the bar code. Similarly to the first reading device 70A and the second reading device 70B, the identification data of the sample read by the third reading device 70C and position data in the sample disk 20 of the sample container 21 to which the identification data is attached are also transmitted to the control device 3, and are stored in the memory 6.

The autonomous analysis device 100 of FIG. 1 has a function of avoiding occurrence of carry-over of the sample and the reagent, that is, a function of reducing or avoiding the carry-over by performing a cleaning operation during measurement. The cleaning operation for avoiding the carry-over can be executed for the pipette nozzles of the first reagent dispensing mechanism 50A, the second reagent dispensing mechanism 50B, and the sample dispensing mechanism 40, and the reaction containers 11.

### <Operation Device>

The operation device 2 is a device operated by an operator and the like when inputting measurement request data (described later) to a computer 7 or causing the monitor 4 to display various types of data. As the operation device 2, a keyboard or a mouse can be typically used, and another operation device such as a touch panel can also be applied.

### <Control Device>

The control device 3 includes an interface 5, a memory 6, the computer 7 (first computer), a control computer 8 (second computer), and a server 9 (third computer).

### -Interface-

The interface 5 is a data input and output unit of the computer 7 for the analysis unit 1. Although the computer 7 and the interface 5 are separately shown in FIG. 1, the interface 5 may be integrated with the computer 7. Data of analysis items sent from the computer 7 to the analysis unit 1 is input to the control computer 8 via the interface 5. In addition, the measurement data by the first measurement unit 60A to the third measurement unit 60C output from the analysis unit 1 via the A/D converter 79 is input to the computer 7 or the memory 6 via the interface 5. The identification data read by the first reading device 70A to the third reading device 70C is also input to the computer 7 or the memory 6 via the interface 5.

### -Memory-

The memory 6 is a storage device such as an HDD or an SSD. FIG. 1 exemplifies an external storage device connected to the computer 7 via the interface 5. However, a storage device built in the computer 7 can also be applied. The memory 6 stores data such as reagent identification information, sample identification information, analysis parameters, measurement request data, calibration results, and measurement data. The measurement request data includes at least a sample ID and a measurement item, and may include other information such as a patient ID as necessary.

### -Computer-

The computer 7 is a control device used by the operator and the like. The computer 7 has functions of creating the measurement request data according to an operation of the operator and the like and outputting the measurement request data to the control computer 8, and displaying and outputting a screen according to the operation of the operator and the like on the monitor 4 based on the measurement data and the like from the analysis unit 1.

Although FIG. 1 exemplifies a configuration in which only one analysis unit 1 is connected to the computer 7, a plurality of analysis units 1 may be connected to one computer 7 via the interface 5. When the plurality of analysis units 1 in a facility are connected, a network (LAN and the like) may be connected to the interface 5, and the plurality of analysis units 1 may be connected to one computer 7 via the network.

### -Control Computer-

The control computer 8 is a control device that outputs an instruction signal to the analysis unit 1 according to the measurement request data received from the computer 7 and instructs analysis. It is assumed that the control computer 8 is integrated with the analysis unit 1 (incorporated inside a body of the analysis unit 1), but the control computer 8 is shown separately from the analysis unit 1 in FIG. 1. Instruction targets of the control computer 8 are mechanisms (operation devices) such as the sample disk 20, the first reagent disk 30A, the second reagent disk 30B, the sample dispensing mechanism 40, the first reagent dispensing mechanism 50A, the second reagent dispensing mechanism 50B, the reaction container transfer mechanism 64, and the reagent dispensing mechanism 67. Although the embodiment is an example in which one control computer 8 collectively drives the analysis unit 1, it is also possible to adopt a configuration in which a dedicated control computer is provided for each operation device and the control computer drives the corresponding operation device according to the input from the computer 7.

### -Server-

The server 9 is connected to the computer 7. Although FIG. 1 shows a configuration in which the computer 7 and the server 9 are connected to each other not via the interface 5, the server 9 may be connected to the computer 7 via the interface 5. In addition, the computer 7 and the server 9 may be connected to each other via the network.

### <Monitor>

The monitor 4 is connected to the computer 7, and is a graphical user interface for operating the computer 7 or a display device for displaying and outputting various types of data. The various types of data displayed and output by the monitor 4 include measurement data by the analysis unit 1, a determination result by the computer 7, patient data, and the like. Desired data is displayed on the monitor 4 according to a signal received from the computer 7 in response to an operation of the operation device 2 by the operator and the like.

### (Basic Operation - Biochemical Test)

An example of a basic operation using the autonomous analysis device 100 will be described. Here, an analysis operation of a measurement item related to a blood coagulation fibrinolysis marker such as a D dimer or an FDP among a biochemical test and a blood coagulation test of the sample using the first spectrophotometer 62A will be described.

Operation parameters for the measurement item that can be analyzed by the autonomous analysis device 100 are input to the computer 7 in advance by the operator and the like and stored in the memory 6. Measurement request data for each sample is input by the operator and the like. When a measurement order of a sample ID to which the measurement request data is input arrives, the operation parameters corresponding to the measurement item of the measurement request data are read from the memory 6 and input from the computer 7 to the control computer 8. Then, the control computer 8 drives the analysis unit 1 according to the operation parameters.

Specifically, in response to an operation instruction from the control computer 8, the reaction disk 10 and the sample disk 20 are first driven to move the target reaction container 11 and the target sample container 21 to the dispensing positions 10a and 20a. Then, a predetermined amount of sample is aspirated from the target sample container 21 at the dispensing position 20a and dispensed into the target reaction container 11 of the reaction disk 10 at the dispensing position 10a by the sample dispensing mechanism 40. The reaction container 11 into which the sample is dispensed is moved from the dispensing position 10a to the dispensing position 10b or 10c by the rotating reaction disk 10, and the reagent corresponding to the measurement item is dispensed by the first reagent dispensing mechanism 50A or the second reagent dispensing mechanism 50B. An order of dispensing the sample and the reagent may be reversed (the reagent is earlier than the sample).

Thereafter, when the reaction container 11 crosses the first measurement unit 60A, the light transmitted through the sample is measured by the first spectrophotometer 62A, the measurement value by the first spectrophotometer 62A is converted into a digital signal by the A/D converter 79, and the digital signal is input to the computer 7 via the interface 5. In the computer 7, a concentration of a mixed solution of the sample and the reagent is calculated as the measurement data based on calibration curve data corresponding to the measurement item and the measurement value. The calibration curve data is measured in advance under a designated analysis method and stored in the memory 6. The measurement data computed by the computer 7 is displayed and output on the monitor 4 according to an operation of the operator and the like or automatically.

The measurement data may be computed by the control computer 8 instead of the computer 7. In the analysis unit 1 of FIG. 1, by using the turntable type reaction disk 10, the sample can be dispensed continuously by a rotation operation of the disk, and a processing capability is excellent.

### (Basic Operation - Blood Coagulation Test)

Another example of the basic operation using the autonomous analysis device 100 will be described. Here, an analysis operation related to measurement of a hemostatic function test item, that is, measurement of a blood coagulation time will be described. In the measurement of the blood coagulation time, the control computer 8 also drives the analysis unit 1 according to the operation parameters.

Specifically, the reaction container 60a accommodated in the reaction container accommodating unit 63 in the third measurement unit 60C is transferred to the sample dispensing station 65 by the reaction container transfer mechanism 64. Then, the sample aspirated from the corresponding sample container 21 of the sample disk 20 is dispensed into the reaction container 60a of the sample dispensing station 65 by the sample dispensing mechanism 40. The reaction container 60a into which the sample is dispensed is transferred to the reaction container temperature control block 66 by the reaction container transfer mechanism 64, and heated to 37°C therein.

On the other hand, the reagent aspirated by the first reagent dispensing mechanism 50A from the first reagent bottle 31A corresponding to the measurement item is discharged to a predetermined empty reaction container 11 disposed in the reaction disk 10. The reagent cooled in the first reagent disk 30A is heated to about 37°C in the reaction disk 10.

After a certain period of time elapses, the reagent kept warm in the reaction container 11 is aspirated by the reagent dispensing mechanism 67 having a reagent heating function, and is further heated to, for example, 40°C inside the reagent dispensing mechanism 67. During this period, the reaction container 60a containing the sample is transferred from the reaction container temperature control block 66 to a predetermined measurement channel 68 by the reaction container transfer mechanism 64. Thereafter, the heated reagent is dispensed into the reaction container 60a of the measurement channel 68 by the reagent dispensing mechanism 67. By this reagent dispensing, a blood coagulation reaction between the sample and the reagent starts inside the reaction container 60a.

After the reagent is discharged in this manner, the measurement values of the light are sequentially output in the measurement channel 68 at predetermined short measurement time intervals (for example, every 0.1 seconds). The output measurement value is converted into a digital signal by the A/D converter 79 and input to the computer 7 via the interface 5. After completion of the photometry, the used reaction container 60a is transferred by the reaction container transfer mechanism 64 and discarded in the reaction container discarding unit 69.

Thus, the computer 7 obtains the blood coagulation time from the measurement value received from the analysis unit 1. Thereafter, a concentration of a mixed solution of the sample and the reagent is computed as the measurement data based on the calibration curve data corresponding to the measurement item and the computed blood coagulation time. The measurement data computed by the computer 7 and the blood coagulation time are displayed and output on the monitor 4 according to an operation of the operator and the like or automatically.

Since the third measurement unit 60C needs to collect the measurement values for a certain period of time, only one reaction container 60a can be measured by one measurement channel 68 in the period. Although a configuration having six measurement channels 68 is exemplified in FIG. 1, when there is no vacancy in the measurement channels 68, the next measurement of the blood coagulation time is not accepted, and the autonomous analysis device is in a standby state. From the viewpoint of preventing the occurrence of the standby state, it is advantageous that the number of measurement channels 68 is large.

### (Function of Control Device)

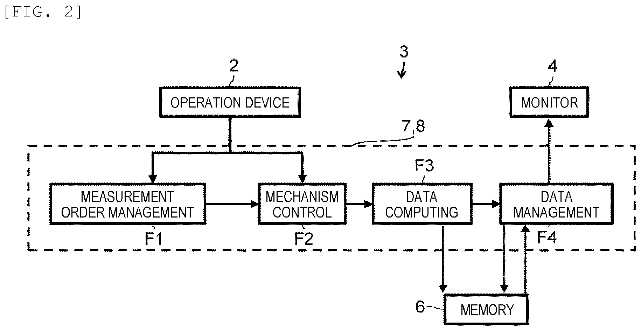

FIG. 2 is a functional block diagram of a control device provided in the autonomous analysis device. In FIG. 2, elements corresponding to those in FIG. 1 are denoted by the same reference numerals as those in FIG. 1, and description thereof will be appropriately omitted. As shown in FIG. 2, the control device 3 has functions of measurement order management F1, mechanism control F2, data computing F3, and data management F4.

These functions are implemented by predetermined processing in cooperation with other hardware by a processor of a computer executing a program. Then, the program for implementing these functions is stored in the memory 6. The program may be provided by being incorporated in the memory 6 in advance, or may be provided by being recorded in a computer-readable recording medium such as a CD-ROM in an installable format file or an executable format file. Further, the program may be downloaded from the computer connected via the network and installed in the memory 6.

In the embodiment, it is assumed that these functions are shared and executed by a plurality of computers, specifically, the functions of the measurement order management F1 and the mechanism control F2 are executed by the control computer 8, and the other two functions are executed by the computer 7. These will be described below. All the functions may be executed by a single computer.

### -Measurement Order Management-

The measurement order management F1 is a function of setting a measurement order of samples. It is assumed that the function of the measurement order management F1 is executed by the control computer 8, and may be executed by the computer 7. The measurement request data set by the operation device 2 in the computer 7 is received from the computer 7 to the control computer 8. For convenience of description, specific measurement request data is referred to as measurement request data X, and a sample whose ID is designated in the measurement request data X is referred to as a sample Y. When the measurement request data X is received from the computer 7, the control computer 8 sets, for the sample Y, a measurement order indicating which sample is to be measured next.

### -Mechanism Control-

The mechanism control F2 is a function of controlling the operation of the analysis unit 1. The function of the mechanism control F2 is executed by the control computer 8. When the measurement order of the sample Y set in the measurement order management F1 comes, the control computer 8 drives the analysis unit 1 to execute measurement on the sample Y. Specifically, the sample Y disposed on the sample disk 20 is dispensed into the reaction container 11 or 60a according to a measurement item designated by the measurement request data X, and is mixed with the reagent according to the measurement item to be reacted as described above.

### -Data Computing-

The data computing F3 is a function of computing measurement data based on the measurement value received from the analysis unit 1. The function of the data computing F3 is executed by, for example, the computer 7. When the sample Y is caused to react with the reagent by the mechanism control F2, photometric values for the sample Y are received from the first measurement unit 60A to the third measurement unit 60C via the A/D converter 79. Measurement data of the designated measurement item for the sample Y is computed based on the measurement values. Here, the computed measurement data for the sample Y is stored in the memory 6 together with the measurement values, the reagent identification information, the sample identification information, the analysis parameters, the measurement request data, the calibration results, and the like.

### -Data Management-

The data management F4 is a function of managing the measurement data. The function of the data management F4 is executed by, for example, the computer 7. Specifically, the computer 7 associates data (measurement data and the like) for the sample Y computed in the data computing F3 with the sample ID of the sample Y. The sample ID of the sample Y is also associated with the measurement data and the like for the sample Y stored in the memory 6 at the same time. Then, the computer 7 automatically or according to an operation by the operator and the like, displays and outputs, to the monitor 4, the measurement data and the like of the sample Y processed by the data management F4. In addition, the measurement data and the like associated with the sample ID of the sample Y is transmitted from the computer 7 to the server 9 and stored in a memory (storage unit) of the server 9. As described above, the memory of the server 9 is a test information database that stores past measurement data. The memory of the server 9 stores an electronic medical chart for each patient ID in addition to the measurement data for each patient ID, and has a role as a patient information database in which various types of data related to an individual patient are collected. The electronic medical chart records findings by a doctor in a medical examination, symptoms of a patient, past medical history, medication history, family medical history, and the like.

### (Example of Confirmation of Coagulation Reaction Course of Target Sample)

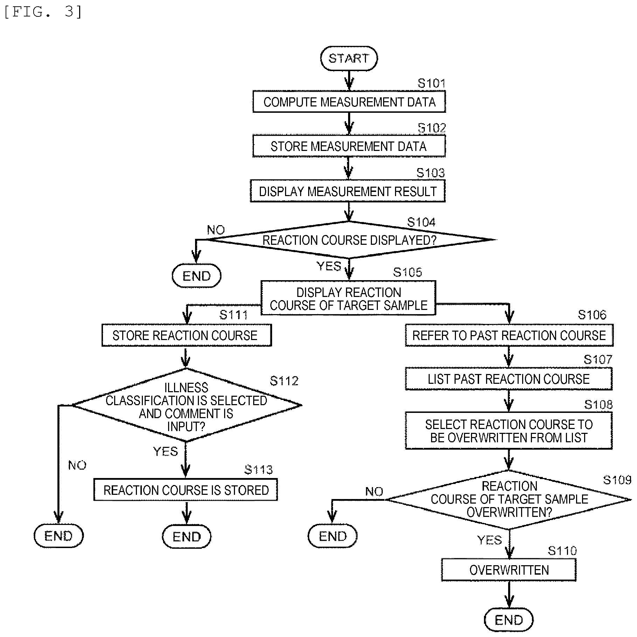

FIG. 3 is a flowchart showing an example of confirmation of the coagulation reaction course of the target sample.

First, in the blood coagulation test designated by the measurement request data, when the measurement data of a target patient ID is output by the computer 7 through processing of the mechanism control F2, confirmation processing of the reaction course shown in FIG. 3 is started. Then, the computer 7 computes the measurement data of the blood coagulation test (step S101), stores a measurement data file of the measurement data of the blood coagulation test in the memory 6 (step S102), and displays a measurement result on the monitor 4 (step S103) .

At this time, when a predetermined operation is performed on a screen of the monitor 4, the coagulation reaction course can be confirmed (step S104). When the predetermined operation is performed in step S104, the computer 7 causes the monitor 4 to display the coagulation reaction course of the target sample (step S105). At this time, a button for referring to the past coagulation reaction course stored in a memory of the autonomous analysis device 100 or a database of the server 9 (hereinafter, referred to as a database and the like) and a button for storing the coagulation reaction course of the target sample in the database and the like are also displayed on the screen of the monitor 4.

Here, when the button for referring to the past coagulation reaction course is selected (step S106), the computer 7 causes the monitor 4 to display a list of the past coagulation reaction course (step S107).

The past coagulation reaction course is classified in advance on an illness basis and stored in the database and the like. When an overwrite button is operated (step S109) in a state in which a predetermined coagulation reaction course classified into a predetermined illness is designated (step S108), the computer 7 overwrites a graph of the designated past coagulation reaction course on a graph of the coagulation reaction course of the target sample (step S110). As described above, not only measurement reaction course information based on measurement data of a sample to be analyzed but also reference reaction course information designated from among past reference reaction course information classified in advance on an illness basis are displayed together. Therefore, the operator and the like can easily find a similar reaction course and easily associate illness patterns.

On the other hand, after step S105, when a button for storing the coagulation reaction course of the target sample is selected (step Sill), the computer 7 causes the monitor 4 to display a screen for selecting a classification (according to an illness) of the stored coagulation reaction course and prompting the operator to input a comment (step S112). Then, when the selection of the classification and the input (omissible) of the comment are completed and a confirmation operation of storage execution is performed, the computer 7 stores the coagulation reaction course of the target sample in a database and the like (step SS113).

### (Example of Screen Display)

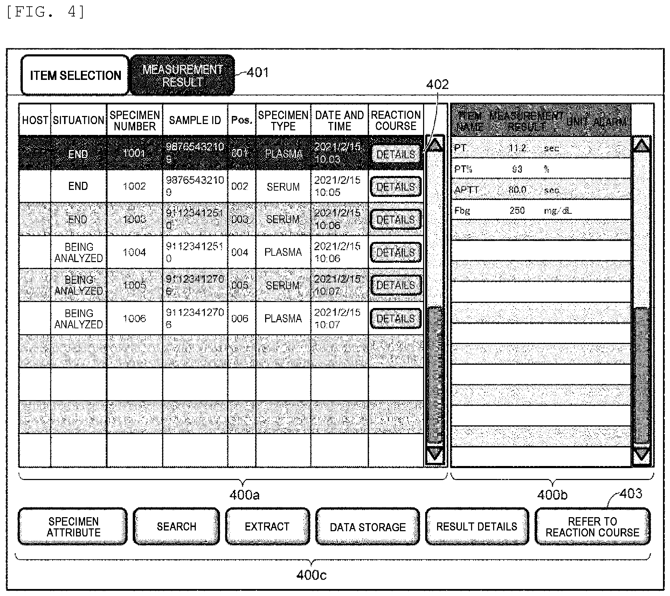

FIG. 4 is a diagram showing an example of a list screen of a measurement result by the autonomous analysis device. Each screen described in the following specification including the screen in FIG. 4 is displayed and output on the monitor 4 by the computer 7 according to the operation of the operator and the like. In addition, an operation including selection of a tab and the like on the screen is performed by the operator and the like using the operation device 2.

The screen in FIG. 4 is displayed when a tab 401 displayed as a "measurement result" is selected (clicked) on a predetermined screen displayed on the monitor 4. The screen in FIG. 4 includes a measurement situation display area 400a for each sample, an item type result display area 400b for displaying a result for each measurement item of the selected sample, and a button display area 400c for each function. A result field of each sample in the measurement situation display area 400a includes a reaction course detail button 402 for viewing a reaction course of an analysis target. In the embodiment, not only the blood coagulation test but also a reaction course of a biochemical and immune test can be stored and referred to. The button display area 400c for each function has a reaction course reference button 403 capable of referring to the past reaction course.

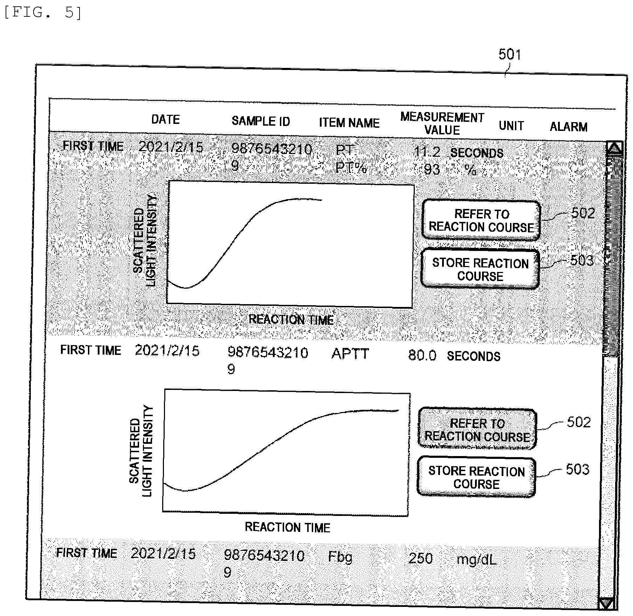

FIG. 5 is a diagram showing an example of the list screen of the coagulation reaction course of the target sample. The screen in FIG. 5 is displayed when the reaction course detail button 402 corresponding to the uppermost sample is selected on the screen in FIG. 4, and a list of the reaction course of the selected sample is displayed in a reaction course list display area 501. A reaction course reference button 502 and a reaction course storage button 503 are displayed on the right side of each reaction course. The example of FIG. 5 shows a state in which the reaction course reference button 502 for comparison is selected with reference to the past reaction course for a measurement item APTT among the selected samples.

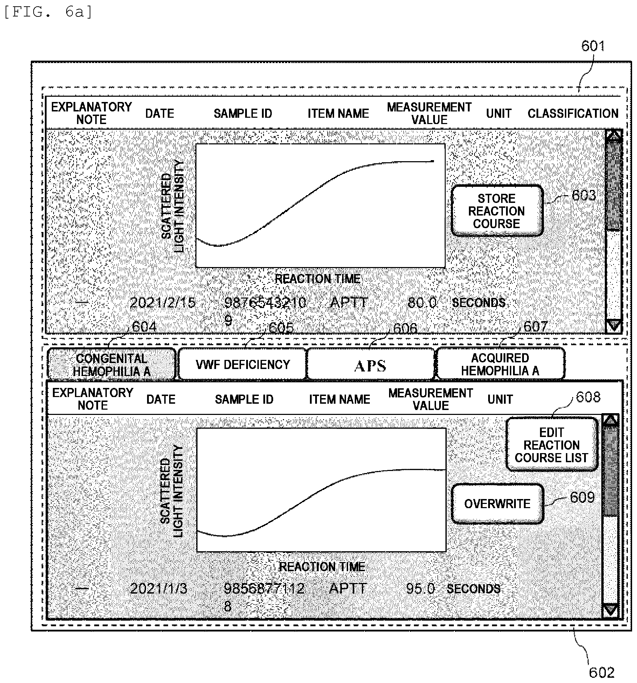

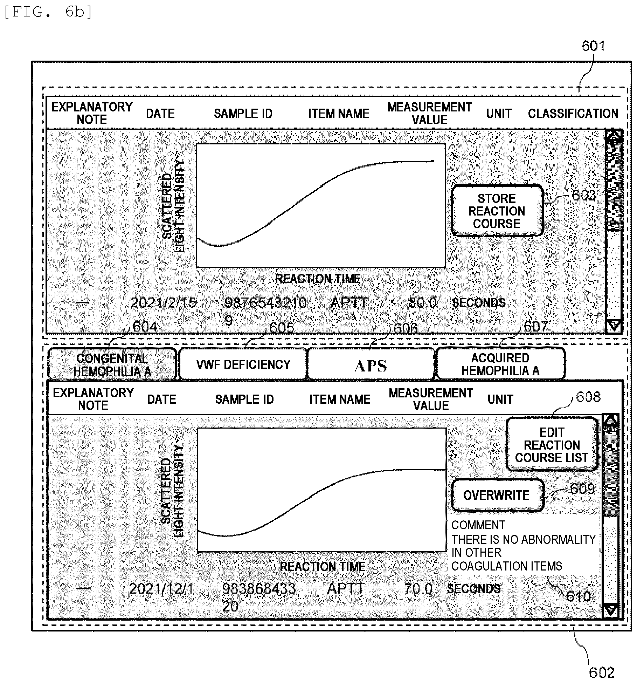

FIG. 6a is a diagram showing an example of a screen on which a coagulation reaction course of the measurement item APTT of the target sample and a past coagulation reaction course of the measurement item APTT classified into a congenital hemophilia A are displayed side by side. The screen in FIG. 6a is displayed when the reaction course is executed after the reaction course reference button 502 corresponding to the measurement item APTT is selected on the screen in FIG. 5. On the screen in FIG. 6, a reaction course display area 601 of the target sample and a past reaction course display area 602 are displayed side by side vertically. The past reaction course of the APTT is stored in the database and the like on an illness basis, and when tabs 604 to 607 are selected (clicked) in the past reaction course display area 602, the past reaction course of the APTT stored on an illness basis is displayed. As will be described later, a display name of the tab can be changed or added by an operation of the operator and the like. In addition, in the past reaction course display area 602, a reaction course list edit button 608 and a reaction course overwrite button 609 are also displayed. In the example of FIG. 6a, since the tab 604 is selected (clicked), in the past reaction course display area 602, a list of the past reaction course stored as the congenital hemophilia A is displayed.

When the reaction course reference button 403 capable of referring to the past reaction course is selected (clicked) on the screen in FIG. 4, a screen for selecting a reaction course of a measurement item to be confirmed is displayed, and then the reaction course of the selected measurement item is displayed.

FIG. 6b is a diagram showing an example of a screen on which the coagulation reaction course of the measurement item APTT of the target sample and a past coagulation reaction course of the measurement item APTT classified into an APS are displayed side by side. The screen in FIG. 6b is displayed when the tab 606 is selected (clicked) on the screen in FIG. 6a. On the screen in FIG. 6b, the past coagulation reaction course of the measurement item APTT classified into the APS is displayed in the past reaction course display area 602. In addition, comments can be input to all reaction courses stored in the database and the like, and the input comments are displayed on a comment display field 610.

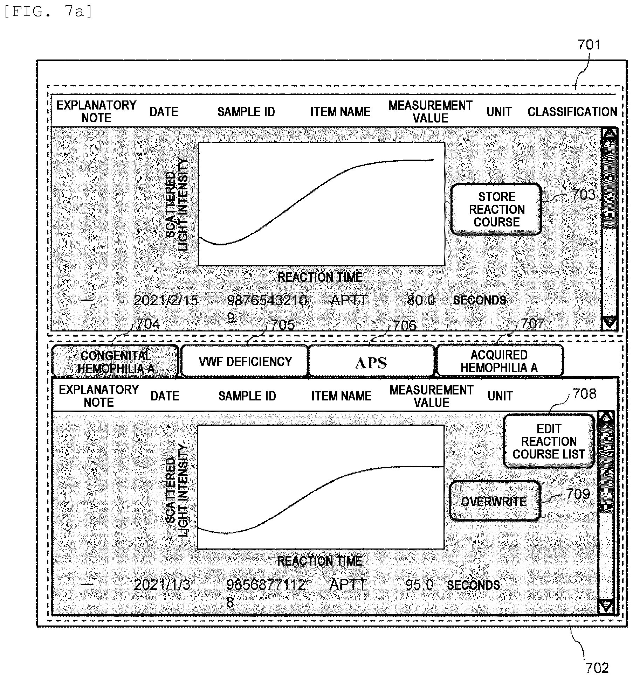

FIG. 7a is a diagram showing a state in which an overwrite button for displaying the past coagulation reaction course in an overlapping manner with the coagulation reaction course of the target sample is selected on the screen in FIG. 6a. As shown in FIG. 7a, in a past reaction course display area 702, since a tab 704, that is, the congenital hemophilia A is selected (clicked), a list of the past reaction course stored as the congenital hemophilia A is displayed, and in this state, a reaction course overwrite button 709 is selected (clicked).

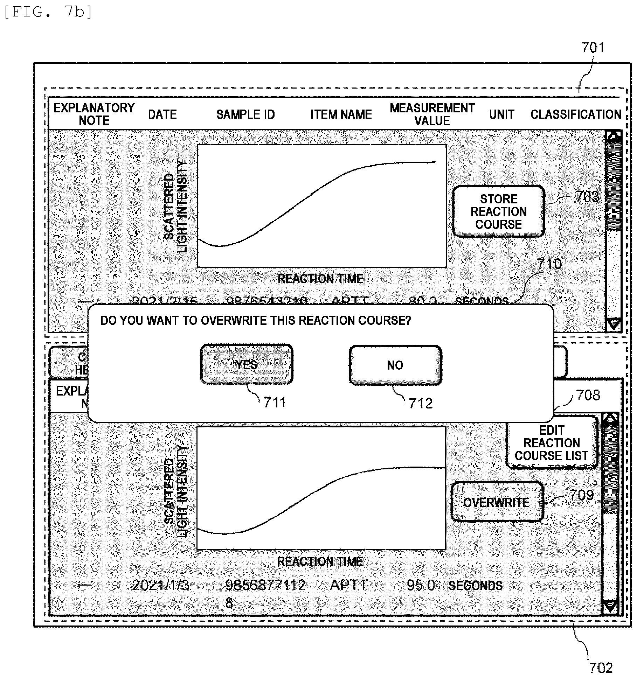

FIG. 7b is a diagram showing an example of a confirmation screen of overwriting, which is displayed after the screen in FIG. 7a. When the reaction course overwrite button 709 is selected (clicked) in FIG. 7a, as shown in FIG. 7b, an overwrite confirmation box 710 is displayed, and an overwrite execution button 711 and an overwrite cancel button 712 are also displayed. In the example of FIG. 7b, the overwrite execution button 711 is selected (clicked).

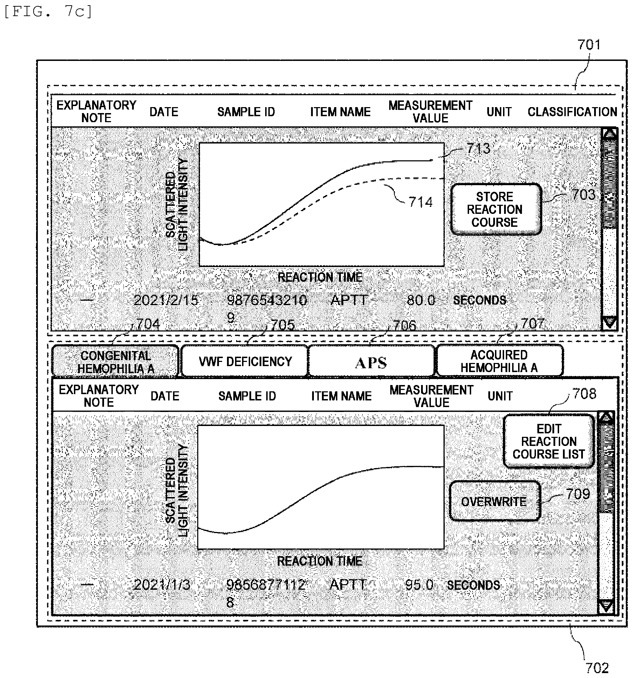

FIG. 7c is a diagram showing an example of a screen after the overwriting is executed, which is displayed after the screen in FIG. 7b. When the overwrite execution button 711 is selected (clicked) on the screen in FIG. 7b, as shown in FIG. 7c, a coagulation reaction course 713 (solid line) of the measurement item APTT of the target sample and a past coagulation reaction course 714 (dotted line) of the measurement item APTT classified into the congenital hemophilia A are displayed in the overlapping manner in the reaction course display area 701 of the target sample. As described above, by displaying a pattern in the overlapping manner, comparison with the pattern of the reaction course corresponding to the illness becomes easier.

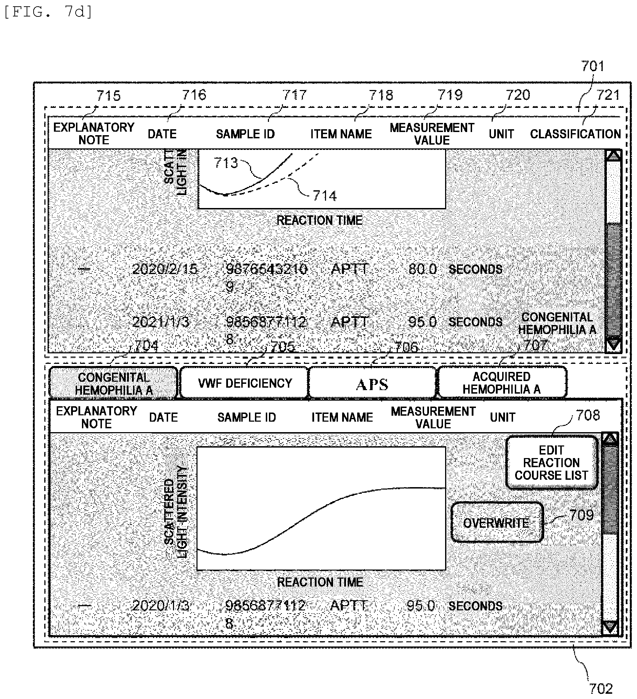

FIG. 7d is a diagram showing an example of a screen when a reaction course display area displaying the overwritten reaction course is scrolled on the screen in FIG. 7c. In the reaction course display area 701 of the target sample, an explanatory note 715, a date 716, a sample ID 717, a measurement item name 718, a measurement value 719, a unit 720, and a classification 721 of a graph are displayed. As shown in FIG. 7d, when the reaction course display area 701 is scrolled, a measurement result and the classification 721 of the overwritten past reaction course are also displayed. Overwriting of the reaction course can be performed a plurality of times, a style of the line is changed such that the reaction course can be easily seen, and the style is displayed in the explanatory note 715 of the graph.

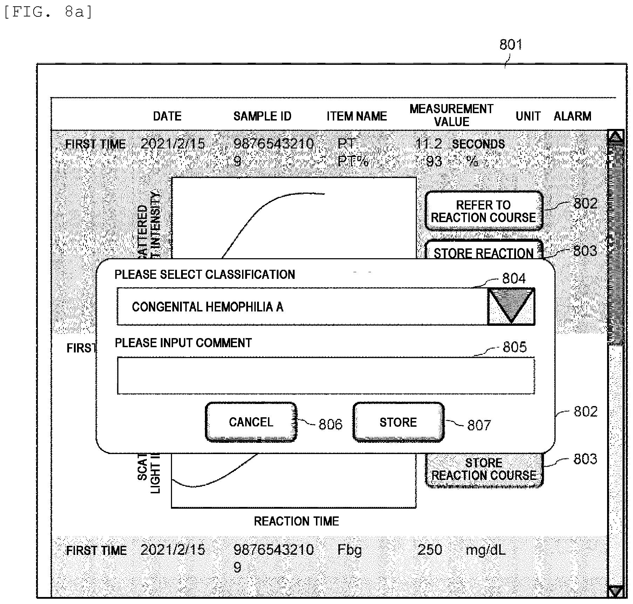

FIG. 8a is a diagram showing an example of a screen for prompting the classification selection and the comment input, which is displayed when the coagulation reaction course of the target sample is stored. The screen in FIG. 8a is displayed after the reaction course storage button corresponding to the measurement item APTT is selected on the screen in FIG. 5. In an illness classification selection box 804, an illness classification to be stored can be selected (clicked), and in a comment input field 805, a comment can be input as necessary. When a storage execution button 807 in a storage cancel button 806 and the storage execution button 807 is selected (clicked), the reaction course of the target sample is stored in the database and the like. When an illness classification of the target sample is already known, the reaction course of the target sample is classified as described above, and the classification result is stored in the database and the like, so that data which can be referred to later as the past reaction course can be expanded.

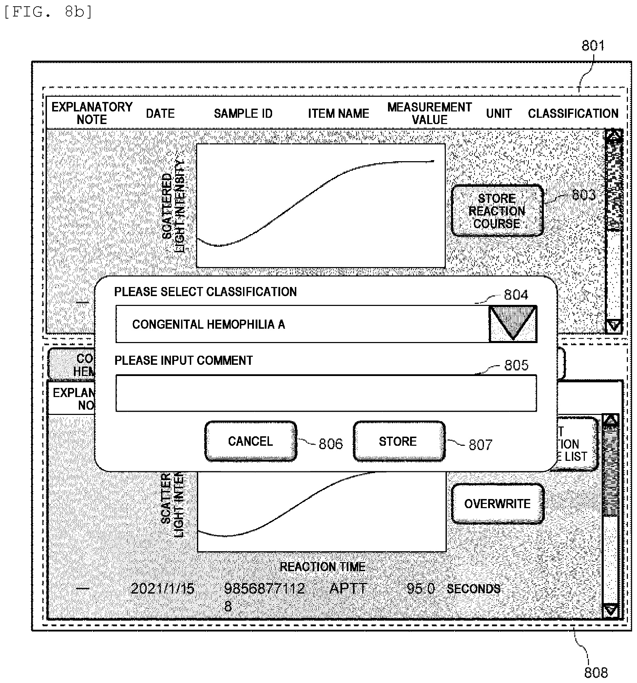

FIG. 8b is a diagram showing another example of the screen for prompting the classification selection and the comment input, which is displayed when the coagulation reaction course of the target sample is stored. The screen in FIG. 8b is displayed after the reaction course storage button in the reaction course display area 701 of the target sample is selected on the screen in FIG. 7a. Similarly to the case of FIG. 8a, in the illness classification selection box 804, the illness classification to be stored can be selected (clicked), and in the comment input field 805, the comment can be input as necessary. In addition, when the storage execution button 807 in the storage cancel button 806 and the storage execution button 807 is selected (clicked), the reaction course of the target sample is stored in the database and the like. As described above, the reaction course of the target sample can be classified with reference to the past reaction course, and the classification result can be stored in the database and the like.

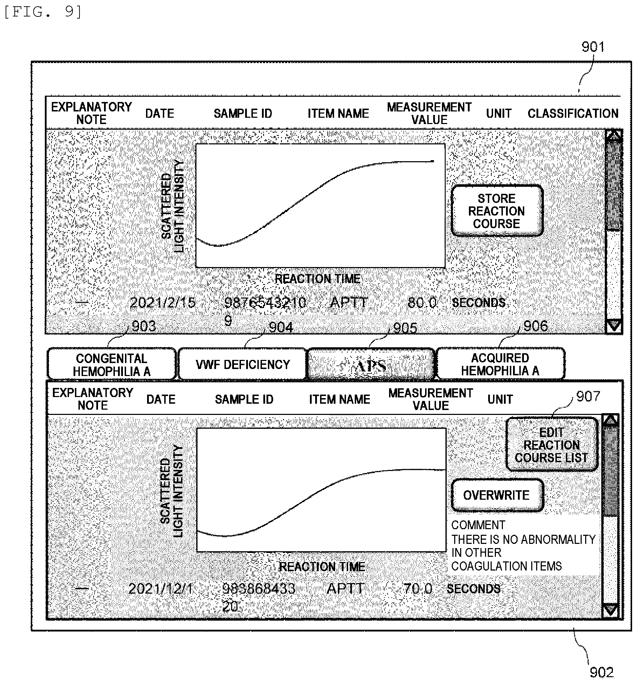

FIG. 9 is a diagram showing a state in which a reaction course list edit button in the past reaction course display area is selected on the screen in FIG. 6b. As shown in FIG. 9, in the past reaction course display area 902, since a tab 905, that is, the APS is selected (clicked), a list of the past reaction course stored as the APS is displayed, and in this state, a reaction course list edit button 907 is selected (clicked).

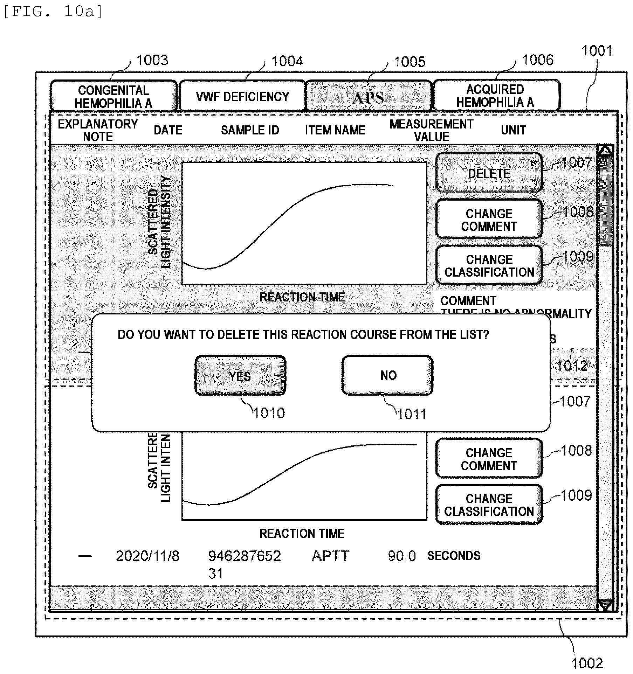

FIG. 10a is a diagram showing an example of a confirmation screen of reaction course deletion, which is displayed when a delete button corresponding to one of the past reaction courses is selected on a screen transitioned from the screen in FIG. 9. In FIG. 9, when a reaction course list edit button in the past reaction course display area is selected (clicked) in a state in which the tab of the APS is selected, a reaction course delete button 1007, a comment change button 1008, an illness classification change button 1009, and a comment display field 1012 (if there is a comment input) are displayed for each of the past reaction courses stored as the APS. In FIG. 10a, only a past reaction course area 1001 and a past reaction course area 1002 immediately before the past reaction course area 1001 are displayed, but when the screen is scrolled, the past reaction course area can also be further confirmed. In addition, the example of FIG. 10a shows a state in which a confirmation box of reaction course deletion is displayed when the reaction course delete button 1007 in the past reaction course area 1001 is selected (clicked). Further, in the example of FIG. 10a, since a delete execution button 1010 is selected (clicked), the target reaction course is deleted.

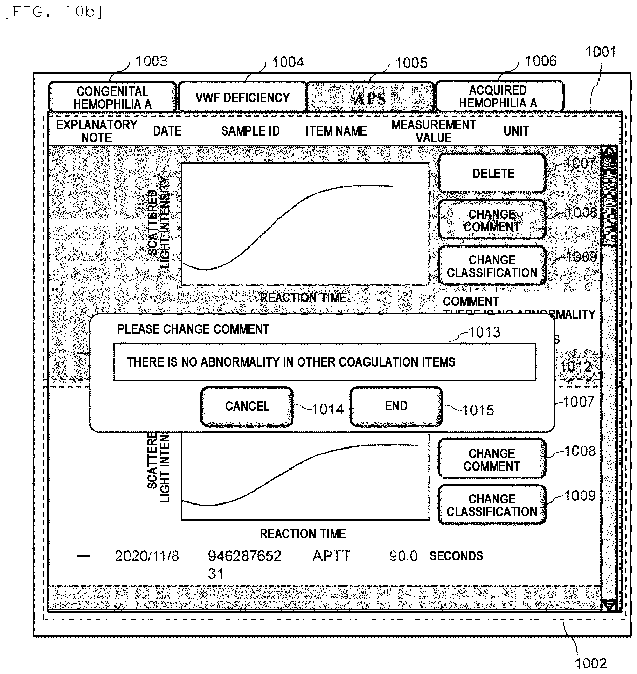

FIG. 10b is a diagram showing an example of a comment change screen displayed when a comment change button corresponding to one of the past reaction courses is selected on the screen transitioned from the screen in FIG. 9. In FIG. 9, when the reaction course list edit button in the past reaction course display area is selected (clicked) in a state in which the tab of the APS is selected, a plurality of buttons for editing the reaction courses are displayed as described above. The example of FIG. 10b shows a state in which a comment change box is displayed when the comment change button 1008 in the past reaction course area 1001 is selected (clicked). Here, when a comment is input to a comment change field 1013 and a comment change end button 1015 is selected (clicked), a comment change is reflected.

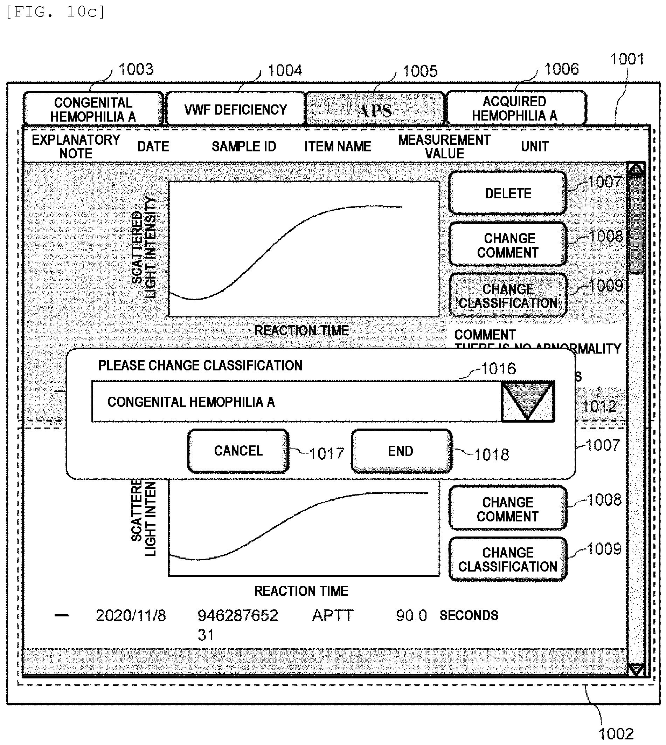

FIG. 10c is a diagram showing an example of a classification change screen displayed when a classification change button corresponding to one of the past reaction courses is selected on the screen transitioned from the screen in FIG. 9. In FIG. 9, when the reaction course list edit button in the past reaction course display area is selected (clicked) in a state in which the tab of the APS is selected, the plurality of buttons for editing the reaction courses are displayed as described above. The example of FIG. 10c shows a state in which an illness classification change box is displayed when the illness classification change button 1009 in the past reaction course area 1001 is selected (clicked). Here, when an illness classification to be changed is selected (clicked) and an illness classification change end button 1018 is selected (clicked), an illness classification change is reflected.

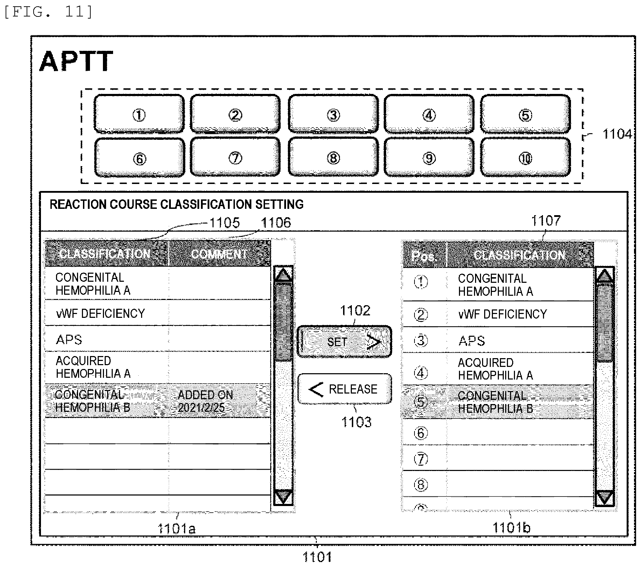

FIG. 11 is a diagram showing an example of an operation screen when keys (tabs) assigned to illness classifications are edited. In a reaction course classification setting area 1101, an illness classification selection area 1101a, a setting button 1102, a release button 1103, and an arrangement selection area 1101b are displayed. In the illness classification selection area 1101a, illness classifications 1105 registered in advance are displayed in a list together with comments 1106 input at the time of registration.

When the illness classifications are assigned to the keys, the operator and the like selects a desired illness classification in the illness classification selection area 1101a, selects one of the keys (for example, key 1 to key 10) to which the selected illness classification is assigned in the arrangement selection area 1101b, and operates (clicks) the setting button 1102. Accordingly, the illness classification selected in the illness classification selection area 1101a is input to an illness classification 1107 in the arrangement selection area 1101b, and the selected illness classification is assigned to a key corresponding to a check box 1104. FIG. 11 shows a state in which since the setting button 1102 is operated (clicked) in a state in which a congenital hemophilia B is selected in the illness classification selection area 1101a and the key 5 is selected in the arrangement selection area 1101b, the congenital hemophilia B is added as the illness classification corresponding to the key 5. In addition, a setting of the key to which the illness classification has already been assigned can be cleared by selecting the key in the arrangement selection area 1101b and operating (clicking) the release button 1103. In the comment area 1106, comments can be freely input.

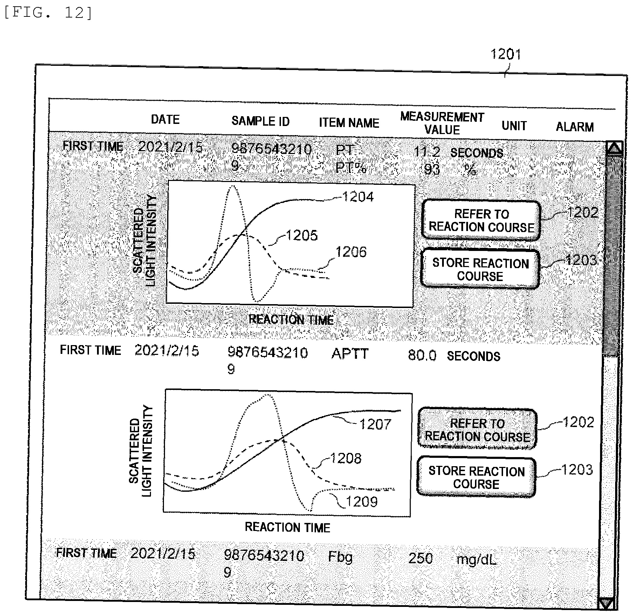

FIG. 12 is a diagram displaying a first derivative curve and a second derivative curve as another example of the list screen of the coagulation reaction course of the target sample. In the example of FIG. 5 described above, only the coagulation reaction course is displayed as the measurement result, but in the example of FIG. 12, not only the coagulation reaction course but also the first derivative curve and the second derivative curve thereof are displayed as the measurement result. For example, regarding a measurement item PT, a coagulation reaction course 1204 is indicated by a solid line, a first derivative curve 1205 of the coagulation reaction course is indicated by a broken line, and a second derivative curve 1206 of the coagulation reaction course is indicated by a dotted line. Similarly, regarding the measurement item APTT, a coagulation reaction course 1207 is indicated by a solid line, a first derivative curve 1208 of the coagulation reaction course is indicated by a broken line, and a second derivative curve 1209 of the coagulation reaction course is indicated by a dotted line. As described above, by displaying not only the reaction course but also the derivative curve thereof, it is possible to accurately determine the tendency of the target sample.

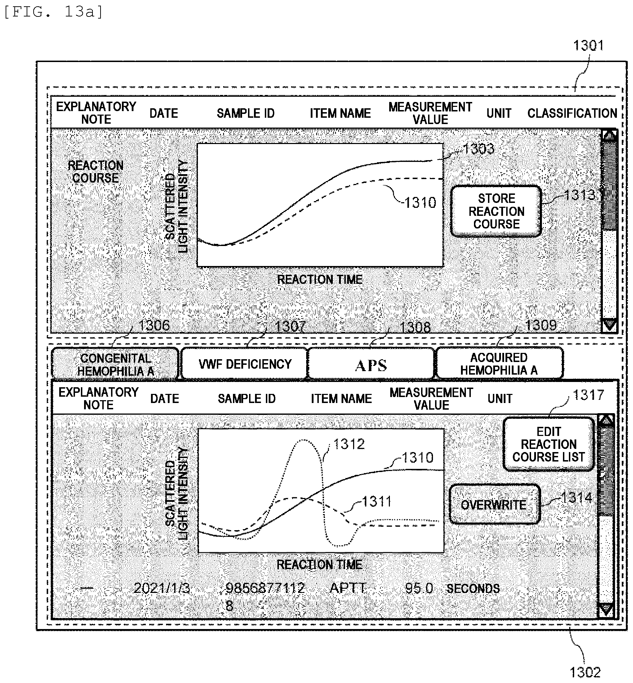

FIG. 13a is a diagram showing an example of a screen when the past coagulation reaction course of the measurement item APTT classified into the congenital hemophilia A is overwritten to the coagulation reaction course of the measurement item APTT of the target sample. In a past reaction course display area 1302, a list of data classified into the congenital hemophilia A can be displayed by scrolling, and not only a coagulation reaction course 1310 (solid line) but also a first derivative curve 1311 (broken line) and a second derivative curve 1312 (dotted line) are displayed in each type of data. In the example of FIG. 13a, since a reaction course overwrite button 1314 corresponding to predetermined data is selected (clicked) in the past reaction course display area 1302, a coagulation reaction course 1303 (solid line) of the measurement item APTT of the target sample and the past coagulation reaction course 1310 (broken line) of the selected measurement item APTT are displayed in an overlapping manner in a reaction course display area 1301 of the target sample.

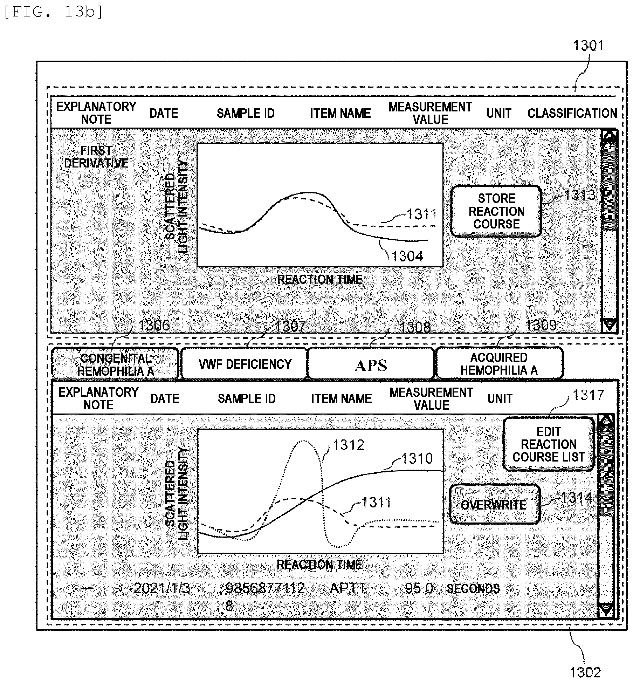

FIG. 13b is a diagram showing the overwritten first derivative curve by scrolling the reaction course display area of the target sample in FIG. 13a. As shown in FIG. 13b, in the reaction course display area 1301 of the target sample, a first derivative curve 1304 (solid line) of the coagulation reaction course of the measurement item APTT of the target sample and the first derivative curve 1311 (broken line) of the past coagulation reaction course of the selected measurement item APTT are displayed in an overlapping manner.

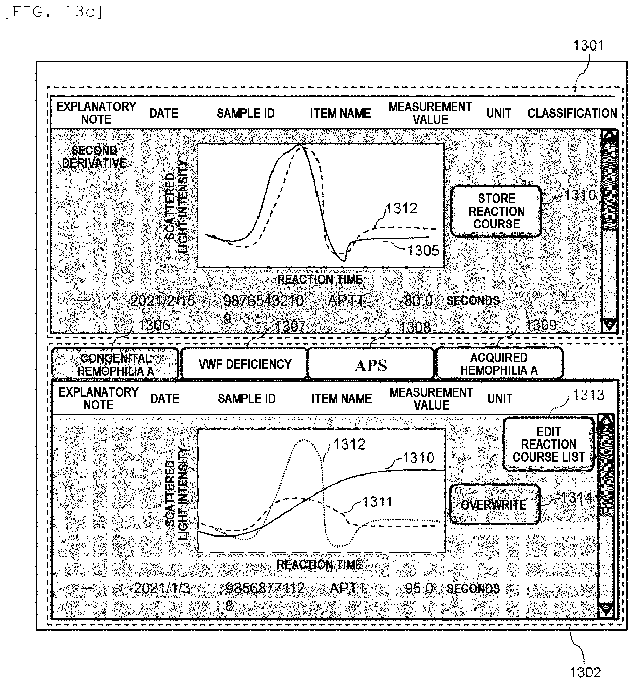

FIG. 13c is a diagram showing the overwritten second derivative curve by further scrolling the reaction course display area of the target sample in FIG. 13b. As shown in FIG. 13c, in the reaction course display area 1301 of the target sample, a second derivative curve 1305 (solid line) of the coagulation reaction course of the measurement item APTT of the target sample and the second derivative curve 1312 (broken line) of the past coagulation reaction course of the selected measurement item APTT are displayed in an overlapping manner.

As described above, the past coagulation reaction course (reference reaction course information) is overwritten on the coagulation reaction course (measurement reaction course information) of the target sample so as to be compared with the coagulation reaction course. Therefore, when an illness of a patient sample is not known, the illness can be predicted to a certain extent based on the coagulation reaction course, and when the illness of the patient sample is known, the illness can be reconfirmed based on the coagulation reaction course. In addition, when the classification of the illness is selected by the operator and the like with respect to the measurement reaction course information, the measurement reaction course information is stored in the database and the like as the reference reaction course information in association with the selected illness. Therefore, by repeating such classification selection, the database is expanded and accuracy of illness prediction is improved.

### (Other Exemplary Embodiments)

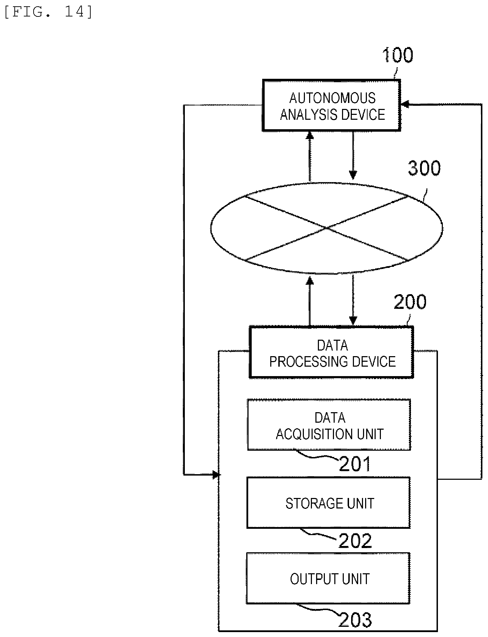

The above-described function (mainly the function of displaying the measurement reaction course information and the reference reaction course information) may be mounted on a data processing device 200 that processes the measurement data of the autonomous analysis device 100 instead of a main body of the autonomous analysis device 100 that analyzes the sample. FIG. 14 is a basic configuration diagram of an analysis system including the autonomous analysis device and the data processing device. As shown in FIG. 14, the autonomous analysis device 100 and the data processing device 200 are connected to each other via a network 300. That is, the measurement data including the measurement reaction course information measured by the autonomous analysis device 100 is transmitted to the data processing device 200, and the past data including the reference reaction course information stored in the data processing device 200 is transmitted to the autonomous analysis device 100. Accordingly, even in the data processing device 200 located away from the autonomous analysis device 100, the above-described display and operation on the screen can be performed. The transmission and reception of data between the autonomous analysis device 100 and the data processing device 200 may be performed directly via a wired or wireless communication line, not via the network 300, or may be performed using an external medium.

The data processing device 200 includes a data acquisition unit 201 that acquires the measurement data from the autonomous analysis device 100, a storage unit 202 that stores the reference reaction course information classified in advance on an illness basis, and an output unit 203 that outputs the measurement reaction course information corresponding to the measurement data and the reference reaction course information corresponding to the selected illness. In addition, the data processing device 200 may be connected to a plurality of autonomous analysis devices 100.

The invention is not limited to the above-described embodiments, and includes various modifications. For example, the above-described embodiments have been described in detail to facilitate understanding of the invention, and the invention is not necessarily limited to those including all the configurations described above. In addition, a part of a configuration according to a certain embodiment can be replaced with a configuration according to another embodiment, and a configuration according to another embodiment can be added to a configuration according to a certain embodiment. In addition, it is possible to add, delete, or replace a part of configurations of each embodiment with another configuration.

### Reference Signs List

1: analysis unit
2: operation device
3: control device
4: monitor
6: memory
7: computer
8: control computer
9: server
10: reaction disk
11: reaction container
12: thermostatic tank
13: stirring mechanism
14: reaction container cleaning mechanism
20: sample disk
21: sample container
30A: first reagent disk
30B: second reagent disk
31A: first reagent bottle
31B: second reagent bottle
40: sample dispensing mechanism
50A: first reagent dispensing mechanism
50B: second reagent dispensing mechanism
60A: first measurement unit
60B: second measurement unit
60C: third measurement unit
60a: reaction container
61A: first light source
61B: second light source
62A: first spectrophotometer
62B: second spectrophotometer
63: reaction container accommodating unit
64: reaction container transfer mechanism
65: sample dispensing station
66: reaction container temperature control block
67: reagent dispensing mechanism
68: measurement channel
69: reaction container discarding unit
70A: first reading device
70B: second reading device
70C: third reading device
79: A/D converter
100: autonomous analysis device
200: data processing device
201: data acquisition unit
202: storage unit
203: output unit
300: network
403, 502, 802, 1202: reaction course reference button
501, 801, 1201: reaction course list display area
503, 603, 703, 803, 1203, 1313: reaction course storage button
601, 701, 901, 1301: reaction course display area of target sample
602, 702, 902, 1302: past reaction course display area
604, 704, 903, 1003, 1306: tab of congenital hemophilia A
605, 705, 904, 1004, 1307: tab of VWF deficiency
606, 706, 905, 1005, 1308: tab of APS
607, 707, 906, 1006, 1309: tab of acquired hemophilia A
608, 708, 907, 1317: reaction course list edit button
609, 709, 1314: reaction course overwrite button
610, 1012: comment display field
713, 1207, 1303: coagulation reaction course of measurement item APTT of target sample
714, 1310: past coagulation reaction course of measurement item APTT
715: explanatory note
716: date
717: sample ID
718: item name
719: measurement value
720: unit
721: classification
1204: coagulation reaction course of measurement item PT of target sample
1205: first derivative curve of coagulation reaction course of measurement item PT of target sample
1206: second derivative curve of coagulation reaction course of measurement item PT of target sample
1208: first derivative curve of coagulation reaction course of measurement item APTT of target sample
1209: second derivative curve of coagulation reaction course of measurement item APTT of target sample
1304: first derivative curve of coagulation reaction course of measurement item APTT of target sample
1305: second derivative curve of coagulation reaction course of measurement item APTT of target sample

## Claims

1. An autonomous analysis device comprising:
an analysis unit configured to analyze a sample;
an operation unit configured to receive an operation from an operator;
a control unit configured to control the analysis unit based on an input from the operation unit and compute measurement data using a measurement value acquired from the analysis unit; and
a display unit configured to display the measurement data computed by the control unit, wherein
the control unit is configured to cause the display unit to display not only measurement reaction course information based on the measurement data of the sample to be analyzed, but also reference reaction course information designated by the operation unit from among reference reaction course information classified in advance on an illness basis.

2. The autonomous analysis device according to claim 1, wherein
the control unit is configured to display the measurement reaction course information and the reference reaction course information side by side or in an overlapping manner on the display unit.

3. The autonomous analysis device according to claim 1, wherein
when a classification of an illness is selected by the operation unit with respect to the measurement reaction course information, the measurement reaction course information is stored as the reference reaction course information in association with the selected illness.

4. The autonomous analysis device according to claim 1, wherein
the control unit is connected to a server that stores the reference reaction course information.

5. A data processing device for processing measurement data of an autonomous analysis device for analyzing a sample, the data processing device comprising:
a data acquisition unit configured to acquire the measurement data from the autonomous analysis device;
a storage unit configured to store reference reaction course information classified in advance on an illness basis; and
an output unit configured to output measurement reaction course information corresponding to the measurement data and reference reaction course information corresponding to a designated illness.

6. A data processing method for processing measurement data of an autonomous analysis device for analyzing a sample, the data processing method comprising:
outputting measurement reaction course information based on measurement data of a sample to be analyzed and reference reaction course information designated from among reference reaction course information classified in advance on an illness basis.

7. A program causing a computer provided in an autonomous analysis device or a computer connected to the autonomous analysis device to execute processing of:
outputting measurement reaction course information based on measurement data of a sample to be analyzed and reference reaction course information designated from among reference reaction course information classified in advance on an illness basis.
